# EUROPEAN PATENT APPLICATION

(11) **EP 4 477 199 A1**
(43) Date of publication of application: **18.12.2024**
(21) Application number: 24180218.0
(22) Date of filing: 05.06.2024
(51) Int. Cl.: A61F 13/15, A61F 13/49, A61F 13/505, A61F 13/532

(54) **A REUSABLE ABSORBENT SANITARY ARTICLE**

(30) Priority: 14.06.2023 IT 202300012195
(71) Applicant: Fameccanica.Data S.p.A., 66020 San Giovanni Teatino (CH) (IT)
(72) Inventor: GUALTIERI, Diego, I-66020 San Giovanni Teatino (Chieti) (IT)
(74) Representative: Buzzi, Notaro & Antonielli d'Oulx S.p.A.

(57) **Abstract**

An absorbent sanitary article (10) comprising a reusable base (12) and a removable absorbent pad (14) detachably carried by an inner surface (30) of the reusable base (12), wherein the reusable base (12) is formed by a sheet of silicone.

## Description

### Field of the invention

The present invention relates to a reusable absorbent sanitary article.

The invention was developed with a view to its application, for example, to baby diapers, absorbent articles for incontinent adults, feminine hygiene articles, etc.

In particular, the invention concerns an absorbent sanitary article including a washable and reusable base or chassis and an absorbent pad carried by the base in a separable manner.

### Description of the prior art

Disposable absorbent sanitary articles have a great impact on municipal solid waste disposal.

It is estimated that absorbent sanitary products constitute approximately 2-3% of total municipal solid waste. However, where recycling collection is carried out with a high percentage of waste differentiation (with a percentage of separated waste exceeding 60% of the total) the percentage of incidence of absorbent sanitary products on the remaining part of the non-separable residual fraction rises to around 20%.

The high incidence of disposable absorbent sanitary products on the residual fraction of non-recyclable waste makes the development of reusable absorbent sanitary products highly desirable.

Washable and reusable absorbent sanitary products are an ecological alternative to traditional disposable products and contribute to a reduction in waste, economic savings, a reduction in environmental impact and the use of more ecological materials.

In general, the trend towards reusable sanitary products is growing and more and more companies are investing in this sector.

In the state of the art there are various solutions of so-called hybrid absorbent sanitary articles, which include a washable and reusable base or chassis and a disposable absorbent pad detachably carried by the base.

US3489149 discloses a menstrual panty having a pocket sewn into the crotch area, the pocket being used to receive a disposable absorbent menstrual pad.

Similarly, US4352356 describes a urinary incontinence panty having a pocket located in the crotch area and configured to receive a disposable urinary incontinence pad.

Another panty-type sanitary garment with a pocket in the crotch area is described in US3613686.

Unlike disposable sanitary articles, which produce a large amount of waste, in absorbent sanitary articles with separable pads the chassis is designed to last a long time and to be washed and reused several times. The quantity of material to be disposed of as waste is reduced by being limited to the separable pad only.

In the state of the art, however, the problem remains of providing absorbent sanitary articles capable of adapting better to the user's body, which take up little space under clothing and which can be washed several times without deteriorating.

### Object and summary of the invention

The object of the present invention is to provide an absorbent sanitary article that satisfies the aforesaid need.

According to the present invention, this object is achieved by an absorbent sanitary article having the characteristics forming the subject of claim 1.

The claims form an integral part of the disclosure provided here in relation to the invention.

### Brief description of the drawings

The present invention will now be described in detail with reference to the attached drawings, given purely by way of non-limiting example, wherein:
- Figure 1 is an exploded perspective view of an absorbent sanitary article according to the present invention,
- Figure 2 is a schematic plan view of the absorbent sanitary article in Figure 1,
- Figure 3 is a schematic plan view of the absorbent sanitary article in Figure 2 with the absorbent pad removed,
- Figures 4 and 5 are schematic cross-sections according to the lines IV-IV and V-V, of Figures 2 and 3,
- Figure 6 is a detail on a larger scale of the part indicated by the arrow VI in Figure 4,
- Figure 7 is an enlarged schematic cross-section of a possible embodiment of a disposable absorbent pad of an absorbent sanitary article according to the present invention,
- Figures 8 and 9 are plan and elevation views of a possible embodiment of a reusable absorbent pad of an absorbent sanitary article according to the present invention,
- Figure 10 is an enlarged view of the detail indicated by the arrow X in Figure 8, and
- Figures 11-13 and 14-16 are views corresponding to Figures 8-10 illustrating the refilling steps of the reusable absorbent pad.

It will be appreciated that the various figures may not be represented on the same scale. It will also be appreciated that some elements or components may not be illustrated to make other elements/components more visible and to simplify the understanding of the figures.

### Detailed description

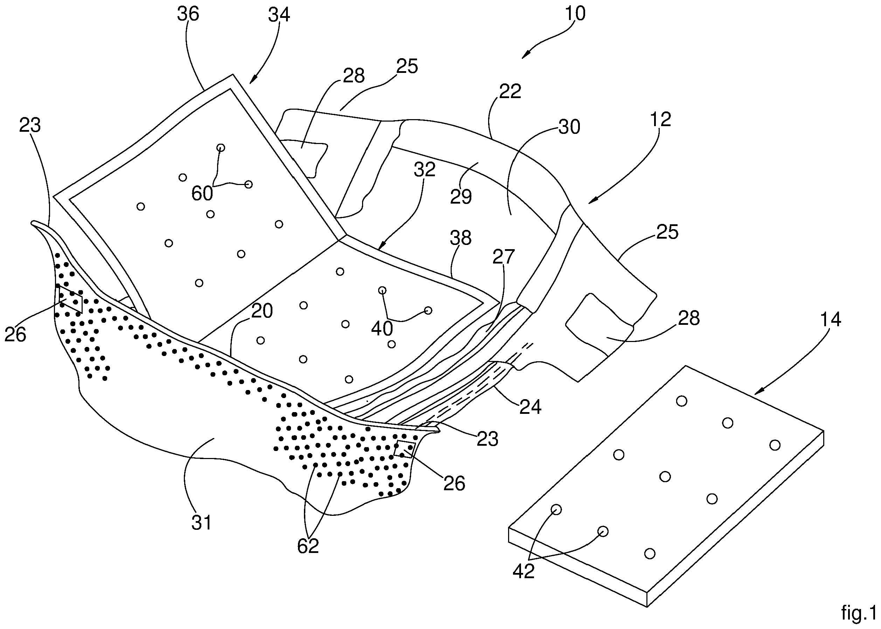

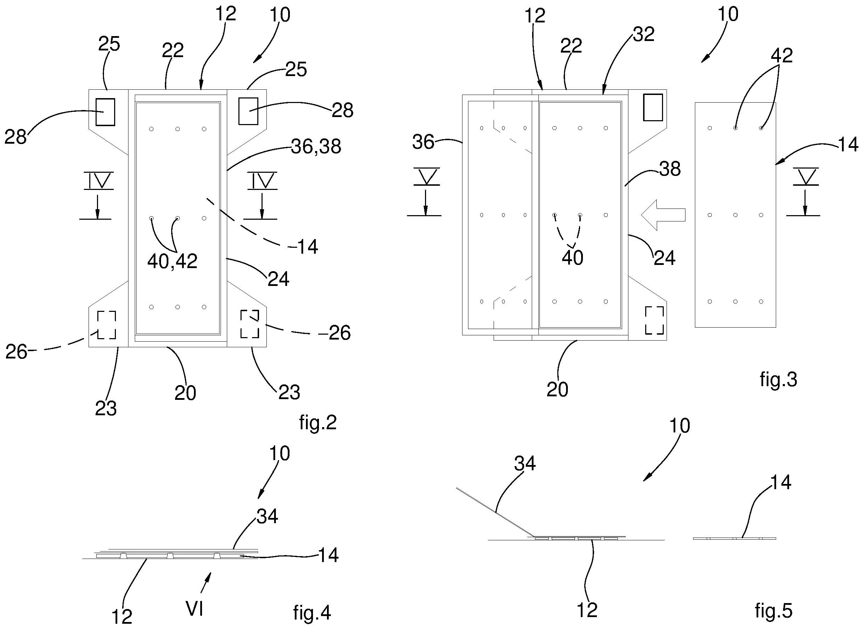

With reference to Figures 1-5, numeral 10 indicates an absorbent sanitary article according to the present invention.

The absorbent sanitary article 10 comprises a reusable base 12 and a removable absorbent pad 14 detachably connected to the reusable base 12.

The removable absorbent pad 14 has the task of absorbing and containing body excretions. The reusable base 12 has the object of containing the removable absorbent pad 14 and allowing the absorbent sanitary article 10 to be wearable.

Absorbent sanitary articles with removable absorbent pads have recently been the subject of growing interest due to their improved sustainability, given the possibility of reusing the base numerous times and significantly reducing the quantity of disposable materials.

The removable absorbent pad 14 itself cannot be worn, as it has no elements that allow it to be closed around the user's waist.

The reusable base 12 has front and rear waist sections 20, 22 that close around the wearer's waist and a crotch section 24 that extends between the front and rear waist sections 20, 22.

According to the present invention, the reusable base 12 is composed of silicone. A reusable base 12 composed of silicone is advantageously characterized by high washability compared to washable and reusable absorbent fabric articles. The high washability gives the reusable base 12 the ability to maintain its characteristics unchanged after one or more washes. The silicone composition of the reusable base 12 allows body excretions to be removed more effectively through a wash cycle compared to washable and reusable fabric absorbent articles, with particular reference to solid and/or semi-solid body excretions.

According to possible advantageous variants, the reusable base 12 is made of medical silicone.

In a possible embodiment, the reusable base 12 may be formed of a sheet of medical silicone. The medical silicone sheet forming the reusable base 12 may have a thickness of between 0.5 and 5 mm.

Medical silicone is a hypoallergenic, resistant and elastic material used in the medical field for producing medical and surgical devices such as, for example, menstrual cups, gloves, cannulas, catheters and other similar products. It is a synthetic material that has high characteristics of flexibility, elasticity, resistance to high temperatures and chemical agents.

Medical silicone is a biocompatible material, therefore, more suitable than silicone for producing absorbent sanitary articles which - during use - are placed in contact with the user's private parts. A material is biocompatible when it does not produce any harmful effects on vital functions and does not cause the onset of allergic reactions or other pathologies.

Biocompatibility is defined by USP class VI (United States Pharmacopoeia), where class VI represents the highest class and establishes the test method.

In particular, regarding the certification process according to USP class VI, this requires the material to be subjected to biological tests, i.e. *In Vivo* Testing, USP <88>. To evaluate the biocompatibility of a material, such as, for example, medical silicone, the tests are based on the administration of samples or extracts of the material on live specimens, such as rabbits or mice. These samples or extracts are obtained from the material by chemical extraction, or rather separation by treatments with solvents, which may include polyethylene glycol or vegetable oil. However, it is customary to begin the process of verifying the biocompatibility of materials with USP <87> *In Vitro* Testing which involves biocompatibility tests on specific cell cultures.

The differences between silicone and medical silicone are substantially in terms of production processes. In fact, to be classified as such, medical silicone must be produced in conditions such as to have no impurity particles incorporated and is, therefore, produced in so-called clean rooms.

The reusable base 12 is elastic, making it comfortable and able to adapt perfectly to the shape of the user's body. The limited thickness of the reusable base 12 allows the creation of an absorbent sanitary article 10 with limited bulk under clothing.

The reusable base 12 has an inner surface 30 which - during use - faces the user and an outer surface 31 which - during use - faces the opposite side of the user, that is towards the user's clothing.

In an extended configuration, the reusable base 12 may have a general hourglass shape. The edges of the crotch section 24 may be arched to conform to the user's legs.

With a view to providing the absorbent sanitary article 10 with suitable breathability to allow the user's skin to breathe adequately, micro-perforations 62 may be formed on the reusable base 12 at least in the front and rear waist sections 20, 22 of the reusable base 12. The micro-perforations may have a diameter between 0.1 and 1 mm. The pattern of the micro-perforations 62 is such that it does not affect the area wherein the removable absorbent pad 14 is located to avoid leaks of liquids through the micro-perforations 62. The pattern of micro-perforations 62 may be characterized by more or less large open areas depending on the required breathability effect.

The front and rear waist sections 20, 22 may be provided with closure formations 26, 28 which allow the reusable base 12 to be closed like a panty around the user's waist.

The closure formations 26, 28 may be openable and refastenable and may be formed by snaps, hooks and loops, surface closures such as Velcro0, or the like. In possible embodiments, the closure formations 26, 28 may be formed integrally in the sheet of silicone or medical silicone forming the reusable base 12.

The front and rear waist sections 20, 22 may have side panels 23, 25 projecting laterally beyond the crotch section 24. The closure formations 26, 28 may be integrally formed or attached to distal portions of the side panels 23, 25.

The absorbent sanitary article 10 may comprise elastic leg cuffs 27 extending to opposite sides of the crotch section 24, integrally formed or attached to the inner surface 30 of the reusable base 12.

The absorbent sanitary article 10 may comprise gasketing elements 29 for containing solid excretions located at the front and rear waist sections 20, 22. The gasketing elements 29 may be integrally formed or attached to the inner surface 30 of the reusable base 12.

The reusable base 12 of medical silicone has a shape comparable to a classic diaper and may be constructed in two ways:
- Ultrasonic or thermal welding of layers with the same principle with which the layers of a disposable diaper are traditionally coupled;
- Formation with 3D printing also known as "Additive Manufacturing", which consists of the process of creating three-dimensional objects through the subsequent deposition layer after layer of material, such as silicone or medical silicone, as shown in the video "Silicone materials for next generation 3D Printing" available at the following address: https://www.youtube.com/watch?v=v8C5P2tXLmY.

The removable absorbent pad 14 is detachably carried by the reusable base 12,

In a possible embodiment, the reusable base 12 comprises an openable pocket 32 located on the inner surface 30 and in which the removable absorbent pad 14 is housed.

The opening pocket 32 may comprise a panel 34. The panel 34 may be integrally formed or attached to the inner surface 30 of the reusable base 12. The panel 34 may be provided with closure formations 36 cooperating with complementary closure formations 38 provided on the inner surface 30 of the reusable base 12 to close the panel 34 around the removable absorbent pad 14.

The panel 34 may be liquid-permeable to allow liquids to penetrate inside the openable pocket 32 where they are absorbed by the removable absorbent pad 14.

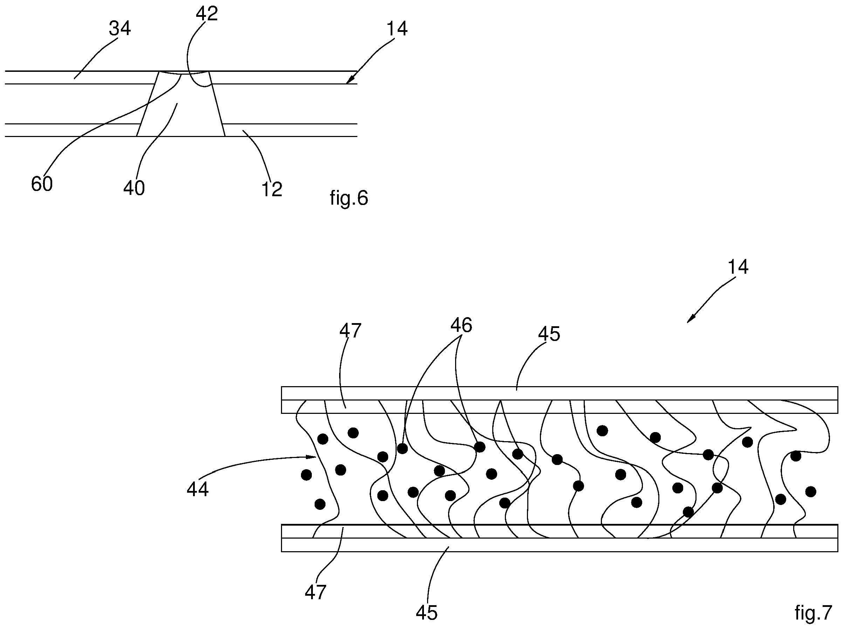

The reusable base 12 may comprise connection formations 40 cooperating with complementary connection formations 42 provided on the removable absorbent pad 14 to hold the removable absorbent pad 14 in a predetermined position relative to the reusable base 12. Referring to Figure 6, the connection formations 40 may be formed by protruding pins formed integrally with the reusable base 12 and the complementary connection formations 42 may be formed by cavities of the removable absorbent pad 14 configured to receive respective pins so as to keep the removable absorbent pad 14 in a pre-established position with respect to the reusable base 12.

The panel 34 may have projections 60 which in the closed position rest on the top of the connection formations 40.

The removable absorbent pad 14 may be disposable or reusable.

If the removable absorbent pad 14 is disposable, when it is saturated with liquid it is removed from the reusable base 12 and replaced with a replacement pad. The reusable base 12 may be washed and reused an indefinite number of times.

The removable absorbent pad 14 - if disposable - may be made of materials commonly used for producing absorbent cores of traditional disposable absorbent sanitary articles, such as: non-woven sheets, Super Absorbent Polymer (SAP), cellulose fluff, Acquisition Distribution Layer (ADL), etc.

The removable absorbent pad 14 may be biodegradable and/or flushable in the toilet.

Figure 7 illustrates a possible embodiment of a disposable removable absorbent pad 14. In the embodiment of Figure 7, the disposable absorbent pad 14 comprises a fiber core 44 in which superabsorbent granular material 46 is incorporated. The superabsorbent granular material 46 is dispersed among the fibers of the fiber core 44. The fiber core 44 may be enclosed between two non-woven sheets 45, which can be attached to the fiber core 44 using layers of glue 47. The fibers forming the fiber core 44 and the superabsorbent granular material 46 may be made of plastic and/or biodegradable material. Such a removable absorbent pad 14 may be produced as described in the document WO2022/034468 by the same applicant.

In possible embodiments, the removable absorbent pad 14 may comprise an absorbent core including cellulose fluff and superabsorbent granular material, which may be sandwiched between two non-woven sheets.

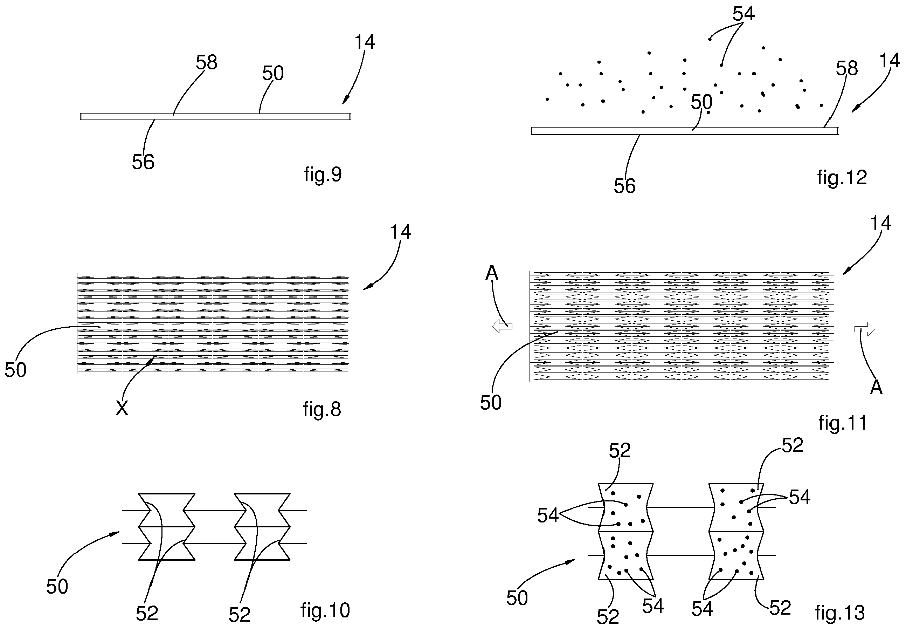

In possible embodiments, the removable absorbent pad 14 may be washable and reusable. With reference to Figures 8-16, in one embodiment the removable absorbent pad 14 may comprise a reticular structure 50 of silicone, or medical silicone, provided with cells 52 that can be filled with superabsorbent granular material 54.

The reticular structure 50 may be located between a silicone base layer 56 and a silicone cover layer 58. The silicone cover layer 58 may be perforated to allow the passage of the superabsorbent granular material 54.

The reticular structure 50 may be auxetic or made with auxetic materials. The term "auxetic" indicates materials or structures characterized by a negative Poisson's coefficient, so that if subjected to traction along a stress direction of, they dilate in the stress direction and in directions transverse to the stress direction. For example, an auxetic structure could expand to occupy a greater volume when subjected to traction and return to its original shape when traction is removed.

Figures 10 and 13 illustrate the reticular structure 50 in a rest configuration and, respectively, in a traction configuration. The reticular structure 50 may comprise a layer of material, such as for example silicone or medical silicone, equipped with cavities configured to form the cells 52.

The auxetic behavior of the reticular structure 50 may be exploited to recharge the cells 52 with superabsorbent granular material 54.

With reference to Figures 11-13, when the reticular structure 50 is subjected to traction along a longitudinal axis A, the cells 52 expand and increase their volume compared to the rest configuration. While the reticular structure 50 is expanded, superabsorbent granular material 54 is poured into the cells 52.

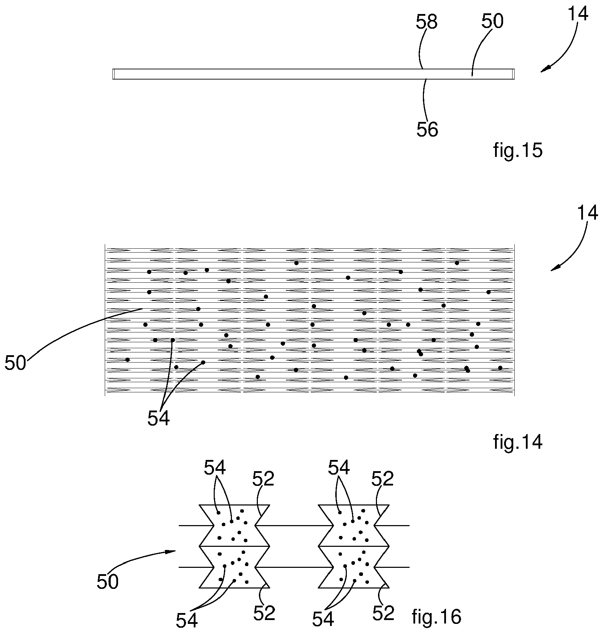

When the traction ceases, the reticular structure 50 returns to its rest configuration as illustrated in Figures 14-16. The shrinkage of the cells 52 is effective in retaining the superabsorbent granular material 54 within the cells 52.

When the superabsorbent granular material 54 contained within the cells 52 is saturated with liquids, the reticular structure 50 is again subjected to traction along the longitudinal axis A to expand the cells and remove the exhausted superabsorbent granular material 50. After the exhausted superabsorbent granular material 50 has been removed from the cells 52 the removable absorbent pad 14 may be washed and refilled with superabsorbent granular material 54.

Of course, without prejudice to the principle of the invention, the details of construction and the embodiments can be widely varied with respect to those described and illustrated, without thereby departing from the scope of the invention as defined by the claims that follow.

## Claims

1. An absorbent sanitary article (10) comprising:
- a reusable base (12) having front and rear waist sections (20, 22) that close around the wearer's waist and a crotch section (24) that extends between the front and rear waist sections (20, 22), and
- a removable absorbent pad (14) detachably carried by the reusable base (12),
**characterized in that** the reusable base (12) is made of silicone and wherein said removable absorbent pad (14) is composed of medical silicone.

2. The absorbent sanitary article (10) according to claim 1, wherein said removable absorbent pad (14) comprises a reticular structure (50) provided with cells (52) and having an auxetic structure such that said cells (52) expand when the reticular structure (50) is subjected to traction along a longitudinal axis (A), and wherein said cells (52) can be filled with superabsorbent granular material (54).

3. The absorbent sanitary article (10) according to claim 1 or claim 2 of the previous claims, wherein said reusable base (12) is composed of medical silicone.

4. The absorbent sanitary article (10) according to any one of the previous claims, wherein the reusable base (12) includes an openable pocket (32) in which said removable absorbent pad (14) is housed.

5. The absorbent sanitary article (10) according to claim 4, wherein said openable pocket (32) comprises a panel (34) integrally formed or fixed to the inner surface (30) of the reusable base (12) and provided with closure formations (36) cooperating with complementary closure formations (38) provided on the inner surface (30) of the reusable base (12).

6. The absorbent sanitary article (10) according to claim 5, wherein said panel (34) is liquid-permeable.

7. The absorbent sanitary article (10) according to any of the preceding claims, wherein at least said front and rear waist sections (20, 22) of said reusable base (12) are breathable.

8. The absorbent sanitary article (10) according to any of the preceding claims, wherein said removable absorbent pad (14) comprises a core of fibers (44) in which superabsorbent granular material (46) is incorporated.
